(19) Europäisches Patentamt
European Patent Office
Office européen des brevets

(11) EP 4 075 439 A1

(12) EUROPEAN PATENT APPLICATION

(43) Date of publication:
19.10.2022 Bulletin 2022/42

(51) International Patent Classification (IPC):
G16H 20/10 (2018.01)

(21) Application number: 22168389.9

(22) Date of filing: 14.04.2022

(52) Cooperative Patent Classification (CPC):
G16H 20/10

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 15.04.2021 US 202163175306 P

(71) Applicant: Insulet Corporation
Acton MA 01720 (US)

(72) Inventors:
• LEE, Joon Bok
  Acton (US)
• ZHENG, Yibin
  Hartland (US)
• O'CONNOR, Jason
  Acton (US)
• ZADE, Ashutosh
  San Diego (US)

(74) Representative: Peterreins Schley
Patent- und Rechtsanwälte PartG mbB
Hermann-Sack-Strasse 3
80331 München (DE)

(54) IMPROVED COMPENSATION FOR CALIBRATION OFFSETS IN AGENT DELIVERY DEVICES

(57) Exemplary embodiments may compensate for loss of calibration by sensors that provide sensed analyte measurements to delivery devices. The exemplary embodiments may obtain one or more calibrated analyte measurements, such as by using techniques to obtain that the analyte measurement value that are known to produce properly calibrated analyte values. The one or more calibrated analyte measurements are compared to a recent analyte measurement to determine a negative or positive offset. The offset may be added to the recent analyte measurement to generate an updated analyte measurement that reflects the calibrated analyte measurement. In addition, past analyte measurement values within a time window may be updated as well by adding the offset to the past analyte measurement values. The exemplary embodiments may also compensate for the past over-delivery or under-delivery of an agent by the delivery device due to the loss of calibration.

FIG. 1A

**Description**

**BACKGROUND**

Delivery devices that deliver agents to users on an ongoing basis often rely on values from sensors to influence their delivery of agents. The values from the sensors may affect when agents are delivered and/or what dosages are delivered. Unfortunately, the sensors may lose their calibration during operation so that the values produced by the sensors are offset by an offset amount relative to true values. This can be problematic in that the delivery devices may rely on the uncalibrated values in determining whether to deliver agents and to determine dosages of the agent to be delivered to the user.

One example of such a delivery device is a delivery device that delivers insulin and/or another agent for altering or controlling blood glucose levels to a user on an ongoing basis. For instance, the delivery device may be an on-body delivery device that remains, to some extent, attached to a user 24 hours a day. The delivery device receives updated blood glucose level values from a glucose monitor on an ongoing basis. When the glucose monitor is no longer properly calibrated, the glucose monitor may produce blood glucose level readings that are offset from true blood glucose levels for the user. These offset blood glucose level readings may be too high or too low. The net result may be that the delivery device either over-delivers or under-delivers the agent to the user. This may result in hypoglycemia or hyperglycemia in some instances or at the very least may produce lower blood glucose levels or higher blood glucose levels than desired.

**SUMMARY**

In accordance with an inventive aspect, a method performed by a processor of an electronic device includes receiving a calibrated value for an analyte measurement of a user of a delivery device and determining a difference between the calibrated value and a recently received analyte measurement that was received from a sensor. The method further includes updating at least some of past analyte measurements received over a past interval from the sensor based on the difference and using the updated past analyte measurements to determine a new dosage of an agent to be delivered to the user by the delivery device.

The analyte measurement may be a measurement of blood glucose. The agent may be one of insulin, glucagon, GLP-1 or another agent for modifying the blood glucose of the user. The agent may include at least one of a chemotherapeutic agent, a pain relief agent, a blood thinner agent, glucagon, a hormonal agent, a pharmaceutical agent or a therapeutic agent. The updating may include updating each of the past analyte measurements that are updated by a same fixed amount. The electronic device may be one of the delivery device or a management device for the delivery device.

In accordance with another inventive aspect, a method performed by a processor of an electronic device incudes receiving a calibrated value for an analyte measurement of a user of a delivery device that delivers dosages of an agent and determining a difference between the calibrated value for the analyte measurement and at least one recently received analyte measurement that was received from a sensor. The method further includes updating at least some of past analyte measurements received over a past interval from the sensor based on the difference and newly determining dosages of an agent that would have been delivered if the updated past analyte measurements were used to determine the dosages rather than the past analyte measurements received from the sensor. The method additionally includes calculating a difference between the newly-determined dosages of the agent and actual dosages of the agent delivered to the user by the delivery device based on past analyte measurements received for the past interval and modifying control for the delivery device to compensate for the calculated difference between the newly-determined dosages of the agent and the actual dosages of the agent.

The agent may be insulin, glucagon, GLP-1, or another agent that modifies blood glucose. The delivery device may be an on-body insulin pump or an insulin pump carried by the user and having an infusion site. The analyte may be blood glucose. The agent may include at least one of a chemotherapeutic agent, a pain relief agent, a blood thinner agent, glucagon, a hormonal agent, a pharmaceutical agent or a therapeutic agent. The calibrated value for the analyte measurement may be a blood glucose level measurement resulting from analyzing blood extracted from the user via a different measurement mechanism than the sensor, e.g., a second blood glucose sensor, such as a fingerstick BG meter. The processor may use insulin on board (IOB) for the user as a factor in determining a dosage of agent to be delivered to the user and wherein modifying the control for the delivery device to compensate for the calculated difference between the newly-determined dosages of the agent and the actual dosages of the agent comprises updating a value of insulin on board for the user by the difference. The insulin action of the insulin on board over a time period may be used to determine the dosage of agent to be delivered to the user. The modifying may modify one or more constraints and wherein the modified one or more constraints remain modified for a predetermined period of time of future operation of the delivery device. The modifying the control for the delivery device to compensate for the calculated difference between the determined dosages of the agent and the actual dosages of the agent may further comprise modifying a value of insulin on board for the user.

In accordance with an additional inventive aspect, a device for controlling deliveries of a delivery device to a user includes a storage for storing a history of agent dosages delivered to a user and a history of analyte measurements for a user received from a sensor and a processor. The processor is configured for: receiving a calibrated value for an analyte measurement of a user of the delivery device; determining a difference between the calibrated value for the analyte measurement and a recently received analyte measurement that was received from a sensor; updating at least some of past analyte measurements received over a past interval from the sensor based on the difference; and using the updated past analyte measurements to determine a new dosage of an agent to be delivered to the user by the delivery device.

The processor may be further configured for: determining dosages of the agent that would have been delivered if the updated past analyte measurements were used to determine the dosages rather than the past analyte measurements received from the sensor; calculating a difference between the determined dosages of the agent and actual dosages of the agent delivered to the user by the delivery device based on past analyte measurements received for the past interval; and modifying control for the delivery device to compensate for the calculated difference between the determined dosages of the agent and the actual dosages of the agent.

The processor may use insulin on board for the user as a factor in determining a dosage of agent to be delivered to the user and wherein modifying the control for the delivery device to compensate for the calculated difference between the determined dosages of the agent and the actual dosages of the agent comprises updating a value of insulin on board for the user by the difference. The analyte measurement may be a measurement of blood glucose.

**BRIEF DESCRIPTION OF THE DRAWINGS**

Figure 1A depicts an illustrative delivery system that is suitable for delivering an agent to a user in accordance with exemplary embodiments.

Figure 1B depicts a block diagram of types of agents that may be delivered by the delivery device.

Figure 2 illustrates a simplified block diagram of an example of such a control loop suitable for practicing exemplary embodiments.

Figure 3 depicts a flowchart of illustrative steps that may be performed by exemplary embodiments in determining what dose of agent to deliver to the user as part of a closed loop control system.

Figure 4A depicts a flowchart of illustrative steps that may be performed by exemplary embodiments to correct the past analyte measurement values.

Figure 4B illustrates an example of using a calibrated analyte level measurement to adjust past analyte level measurements in accordance with exemplary embodiments.

Figure 4C shows past analyte level concentration measurements adjusted by an offset in accordance with exemplary embodiments.

Figure 5 depicts a flowchart of illustrative steps that may be performed to obtain a calibrated analyte level measurement where the analyte is blood glucose in accordance with exemplary embodiments.

Figure 6A depicts a flowchart of illustrative steps that may be performed in exemplary embodiments to compensate for the over-delivery or under-delivery of the agent.

Figure 6B shows an example of insulin delivery when constraints are modified in accordance with exemplary embodiments.

Figure 7 depicts a flowchart of illustrative steps that may be performed by exemplary embodiments in adjusting the IOB constraint.

Figure 8 depicts a flowchart of steps that may be performed to determine $U_{diff}(i)$.

Figure 9 depicts a table of values that help illustrate the effects of the adjustments to past blood glucose concentration values and adjustments to the IOB constraint to compensate for past over-delivery or under-delivery of insulin in exemplary embodiments.

**DETAILED DESCRIPTION**

Exemplary embodiments may compensate for loss of calibration by sensors that provide sensed analyte measurements to delivery devices. Sensors may lose calibration or accuracy for a number of reasons, including age of the sensor (for example, may become less accurate as the sensor approaches its end of life), time since sensor was applied to the body (for example, a continuous glucose monitor attached to the body may be less accurate on the first day the sensor begins taking readings), how securely the sensor is attached to the body (for example, a continuous glucose monitor may produce inaccurate readings if it comes loose from the body), forces on the sensor while it is making readings (for example, if a continuous glucose monitor is compressed against the body while the user is sleeping, it may detect less glucose than it otherwise would under normal operation conditions). The exemplary embodiments may obtain one or more calibrated analyte measurements, such as by using techniques to obtain the analyte measurement value that are known to produce properly calibrated analyte values, such as a fingerstick blood glucose meter, which are known to provide more accurate results than a continuous glucose meter worn on the body. The one or more calibrated analyte measurements are compared to a recent analyte measurement to determine a negative or positive offset. The offset may be added to the recent analyte measurement to generate an updated analyte measurement that reflects the calibrated analyte measurement. In addition, past analyte measurement values within a time window may be updated as well by adding the offset to the past analyte measurement values within the time window. This corrects the loss of calibration for those past analyte measurement values and ensures that the history of analyte measurement values relied upon by the control approach of the delivery device has better calibrated values to produce better agent delivery dosages.

The exemplary embodiments may also compensate for the past over-delivery or under-delivery of an agent by the delivery device due to the loss of calibration. The exemplary embodiments may determine the magnitude of the over-delivery or under-delivery of the agent resulting from the loss of calibration over a preceding interval. The exemplary embodiments may relax or tighten one or more constraints of the control approach of the delivery device, or modify a delivery pattern (e.g., a basal delivery rate) to compensate for the determined amount of over-delivery or under-delivery of the agent during the interval. For example, where the agent is insulin and the insulin was under-delivered over the interval due to the loss of calibration, the one or more constraints may be relaxed to deliver more insulin over a period of time until the over-delivery has been addressed. Similarly, where the agent is insulin and the insulin was over-delivered, the one or more constraints may be tightened to deliver less insulin over the period of time until the under-delivery has been addressed. The one or more constraints may revert to their previous values after the compensation is complete.

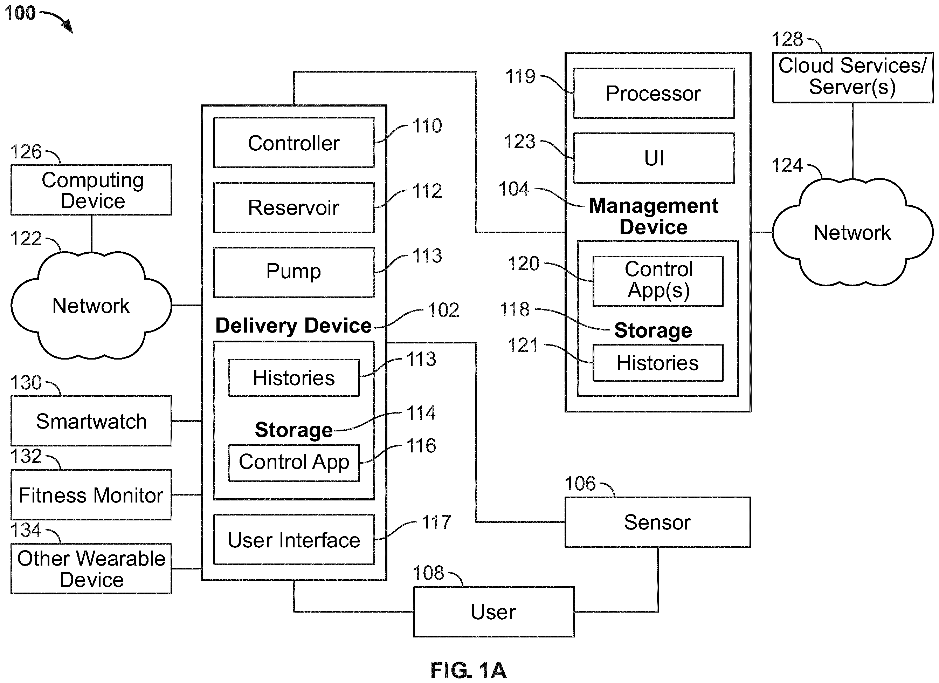

Figure 1A depicts an illustrative delivery system 100 that is suitable for delivering an agent to a user 108 in accordance with exemplary embodiments. The delivery system 100 includes a delivery device 102. The delivery device 102 may be a wearable device that is worn on the body of the user 108 or carried by the user and having an infusion site. The delivery device 102 may be directly coupled to a user (e.g., directly attached to a body part and/or skin of the user 108 via an adhesive or the like) or carried by the user (e.g., on a belt or in a pocket) with tubing connecting the delivery device 102 to an infusion site where the agent is injected. In a preferred embodiment, a surface of the delivery device 102 may include an adhesive to facilitate attachment to the user 108.

The delivery device 102 may include a controller 110. The controller 110 may be implemented in hardware, software, or any combination thereof. The controller 110 may, for example, be a microprocessor, a logic circuit, a field programmable gate array (FPGA), an application specific integrated circuit (ASIC) or a microcontroller coupled to a memory. The controller 110 may maintain a date and time as well as other functions (e.g., calculations or the like). The controller 110 may be operable to execute a control application 116 stored in the storage 114 that enables the controller 110 to direct operation of the delivery device 102. The control application 116 may control delivery of an agent to the user 108 per a control approach like that described herein. The storage 114 may hold histories 113 for a user, such as a history of basal deliveries, a history of bolus deliveries, and/or other histories, such as a meal event history, exercise event history and/or the like. In addition, the controller 110 may be operable to receive data or information. The storage 114 may include both primary memory and secondary memory. The storage may include random access memory (RAM), read only memory (ROM), optical storage, magnetic storage, removable storage media, solid state storage or the like.

The delivery device 102 may include one or more housings for housing its various components including a drive system, a power source, and a reservoir 112 for storing an agent for delivery to the user 108 as warranted. A fluid path to the user 108 may be provided, and the delivery device 102 may expel the agent from the reservoir 112 to deliver the agent to the user 108 via the fluid path. The fluid path may, for example, include tubing coupling the delivery device 102 to the user 108 (e.g., tubing coupling a cannula to the reservoir 112), and may include tubing to a separate infusion site.

There may be one or more communications links with one or more devices physically separated from the delivery device 102 including, for example, a management device 104 of the user and/or a caregiver of the user and/or a sensor 106. The communication links may include any wired or wireless communication links operating according to any known communications protocol or standard, such as Bluetooth®, Wi-Fi, a near-field communication standard, a cellular standard, or any other wireless protocol. The delivery device 102 may also include a user interface 117, such as an integrated display device for displaying information to the user 108 and in some embodiments, receiving information from the user 108. The user interface 117 may include a touchscreen and/or one or more input devices, such as buttons, knobs, or a keyboard.

The delivery device 102 may interface with a network 122. The network 122 may include a local area network (LAN), a wide area network (WAN) or a combination therein. A computing device 126 may be interfaced with the network, and the computing device may communicate with the delivery device 102.

The delivery system 100 may include a sensor 106 for sensing the levels of one or more analytes. The sensor 106 may be coupled to the user 108 by, for example, adhesive or the like and may provide information or data on one or more medical conditions and/or physical attributes of the user 108. The sensor 106 may, in some exemplary embodiments provide periodic blood glucose concentration measurements and may be a continuous glucose monitor (CGM), or another type of device or sensor that provides blood glucose measurements. Additionally or alternatively, the sensor 106 may be physically separate from the delivery device 102 or may be an integrated component thereof. The sensor 106 may provide the controller 110 with data indicative of measured or detected blood glucose levels of the user 108. The sensor 106 alternatively may measure things such as blood pressure, heart rate, blood alcohol, galvanic skin response, temperature, amount of an analyte in blood or in interstitial fluid. The information or data provided by the sensor 106 may be used to adjust delivery operations of the delivery device 102.

The delivery system 100 may also include a management device 104. In some embodiments, no management device is needed as delivery device 102 may manage itself. The management device 104 may be a special purpose device, such as a dedicated personal diabetes manager (PDM) device. The management device 104 may be a programmed general-purpose device, such as any portable electronic device including, for example, a dedicated controller, such as processor, a micro-controller, or the like. The management device 104 may be used to program or adjust operation of the delivery device 102 and/or the sensor 104. The management device 104 may be any portable electronic device including, for example, a dedicated device, a smartphone, a smartwatch or a tablet. In the depicted example, the management device 104 may include a processor 119 and a storage 118. The processor 119 may execute processes to manage a user's blood glucose levels and to control the delivery of the agent to the user 108. The processor 119 may also be operable to execute programming code stored in the storage 118. For example, the storage may be operable to store one or more control applications 120 for execution by the processor 119. The one or more control applications 120 may be responsible for controlling the delivery device 102, such as by controlling the AID delivery of insulin to the user 108. The storage 118 may store the one or more control applications 120, histories 121 like those described above for the delivery device 102, and other data and/or programs.

The management device 104 may include a user interface (UI) 123 for communicating with the user 108. The user interface 123 may include a display, such as a touchscreen, for displaying information. The touchscreen may also be used to receive input when it is a touch screen. The user interface 123 may also include input elements, such as a keyboard, button, knobs, or the like.

The management device 104 may interface with a network 124, such as a LAN or WAN or combination of such networks. The management device 104 may communicate over network 124 with one or more servers or cloud services 128.

Other devices, like smartwatch 130, fitness monitor 132 and wearable device 134 may be part of the delivery system 100. These devices may communicate with the delivery device 102 to receive information and/or issue commands to the delivery device 102. These devices 130, 132 and 134 may execute computer programming instructions to perform some of the control functions otherwise performed by controller 110 or processor 119. These devices 130, 132 and 134 may include displays for displaying information. The display may show a user interface for providing input by the user, such as to request a change or pause in dosage or to request, initiate, or confirm delivery of a bolus of an agent, or for displaying output, such as a change in dosage (e.g., of a basal delivery amount) as determined by controller 110 or management device 104. These devices 130, 132 and 134 may also have wireless communication connections with the sensor 106 to directly receive analyte measurement data.

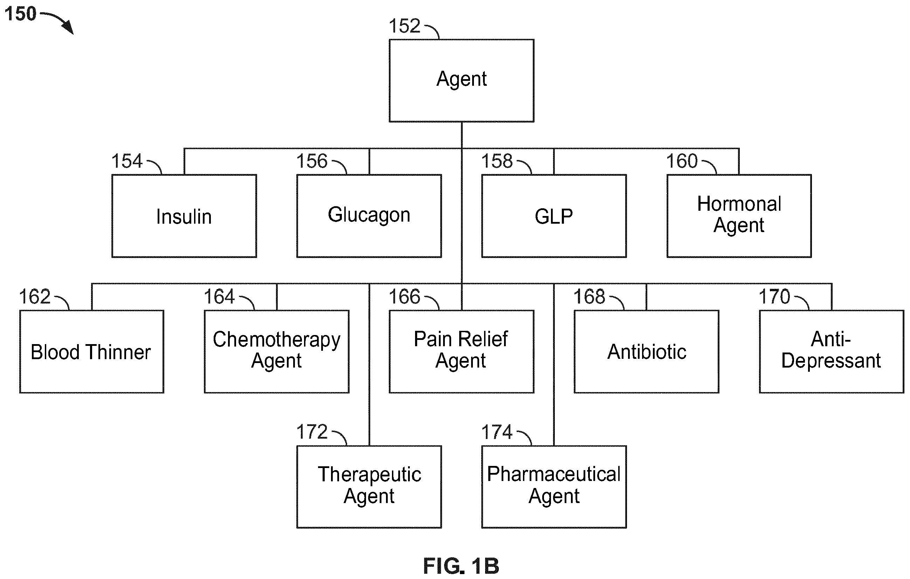

A wide variety of agents may be delivered by the delivery device 102. As shown in Figure 1B, the agent 152 may be insulin 154 for treating diabetes. The agent 152 may be glucagon for raising a user's blood glucose level. The agent 152 may also be a glucagon-like peptide (GLP)-1 receptor agonists for lowering blood glucose or slowing gastric emptying, thereby delaying spikes in blood glucose after a meal. The agent 152 may be a hormonal agent 160, like estrogen, testosterone or the like. The agent 152 may be a blood thinner 162 or a chemotherapy agent 164. The agent 152 may be a pain relief agent 166, an antibiotic 168 or an anti-depressant 170. In general, the agent 152 may be a therapeutic agent 172 or a pharmaceutical agent 174.

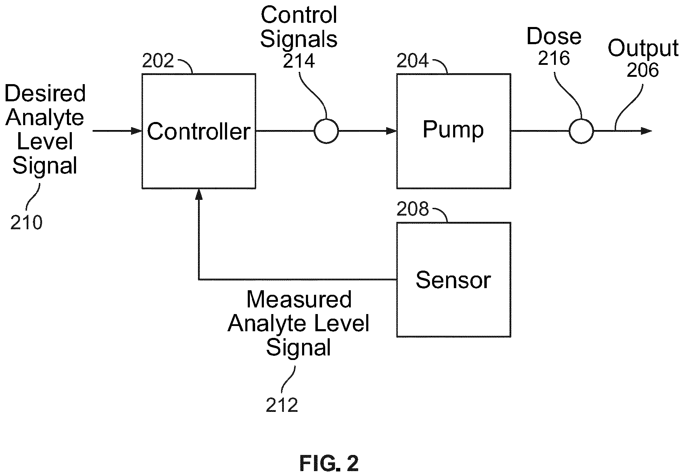

A control loop may be provided to adjust the basal delivery dosage based on current analyte measurements (e.g., blood glucose level readings). Figure 2 illustrates a simplified block diagram of an example of such a control loop 200 suitable for practicing an exemplary embodiment. The example control loop 200 may include a controller 202, a pump mechanism or other fluid extraction mechanism 204 (hereinafter "pump 204"), and a sensor 208. The controller 202, pump 204, and sensor 208 may be communicatively coupled to one another via wired or wireless communication paths. The controller may be part of the delivery device or part of a management device or other device. The sensor 208 may be in some embodiments a glucose monitor, such as a continuous glucose monitor (CGM).

As shown in the example, the controller 202 may receive a desired analyte level signal 210, indicating, for example, a desired blood glucose level or range for a user. The desired analyte level signal 210 may be received from a user interface to the controller or other device, or by an algorithm that automatically determines a desired analyte level for a user. The sensor 208 (or 106) may be coupled to the user and be operable to measure an approximate value of an actual analyte level value for the user. The measured analyte level values are only approximate values of the actual analyte level values for the user, and it should be understood that there may be errors in the measured analyte level values. The errors may, for example, be attributable to a number of factors such as age of the sensor 208, location of the sensor 208 on a body of a user, environmental factors (e.g., altitude, humidity, barometric pressure), or the like. The sensor 208 generates a measured analyte level signal 212.

Based on the desired analyte level signal 210 and the measured analyte level signal 212, the controller 202 may generate one or more control signals 214 for directing operation of the pump 204. For example, one of the control signals 214 may cause the pump 204 to deliver a dose of an agent 216 to a user via output 206. The dose of agent 216 may, for example, be determined based on a difference between the desired analyte level signal 210 and the measured analyte level signal 212 for each measurement cycle or a collection of measurement cycles. The dose of agent 216 may be determined as an appropriate amount of agent to drive the measured analyte level of the user to the desired analyte level. Based on operation of the pump 204 as determined by the control signals 214, the user may receive the dose of agent 216 from the pump 204.

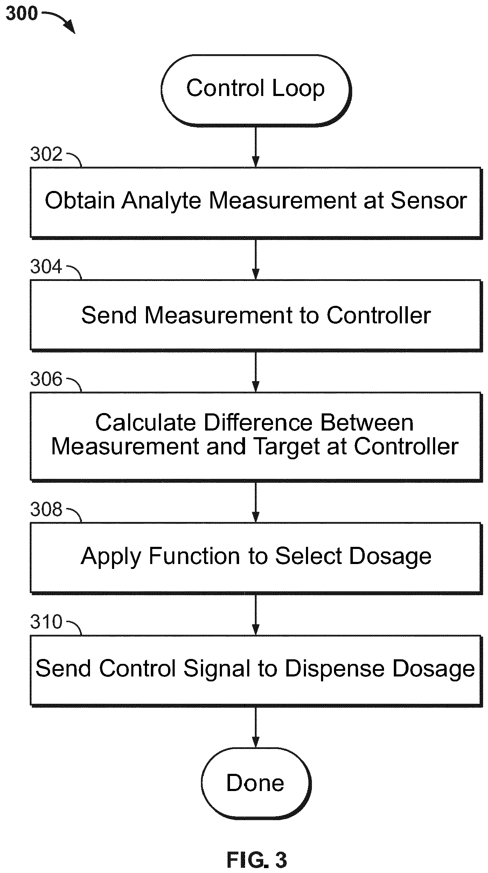

Figure 3 depicts a flowchart 300 of illustrative steps that may be performed by exemplary embodiments in determining what dose of agent to deliver to the user as part of the closed loop control system. These steps may be performed by controller 110, processor 119 on management device 104, or other components (at least in part), like smartwatch 130, fitness monitor or wearable device 134. That said, for purposes of simplicity below, reference will be made to the case where the controller 110 alone controls the functionality. Initially, as was described above relative to Figure 2, a measured analyte level is obtained by the sensor 208 at step 302. The measured analyte level is sent via a signal 212 to the controller 202 at step 304. The controller 202 calculates a difference between the measured analyte level and the desired analyte level at step 306. The closed loop control system may attempt to minimize the aggregate penalty of a cost function over a wide range of possible dosages. The cost function is applied to the possible dosages, and the dosage with the best cost function value is selected at step -308. Depending on how the cost function is configured, the best value may be the lowest value or the highest value. A control signal 214 may be generated by the controller 202 and sent to the pump 204 to cause the pump to deliver the desired dose of the agent 216 to the user at step 310-.

As was mentioned above, one of the difficulties is that the sensor 106 may tend to lose their calibration and/or provide inaccurate measurements during operation. The exemplary embodiments provide a way to recalibrate and account for prior data from the sensor being inaccurate. Moreover, the exemplary embodiments may account for the over-delivery or under-delivery of the agent due to the loss of calibration or provision of inaccurate measurements.

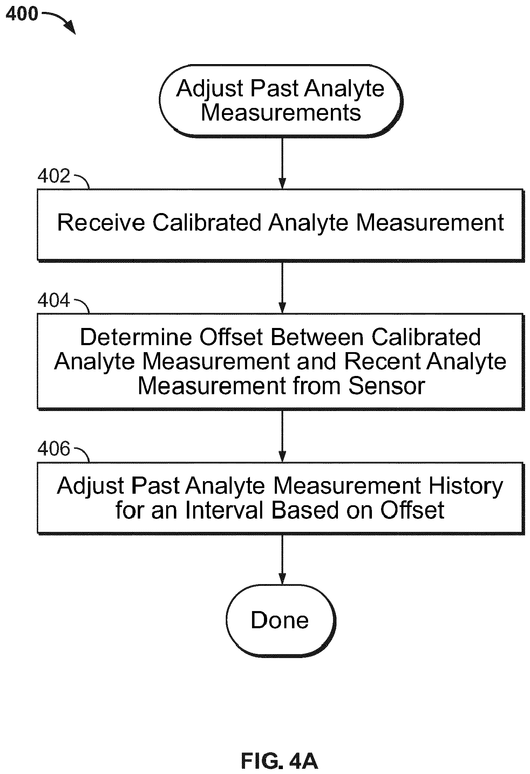

The exemplary embodiments may use a calibrated analyte level measurement to update past analyte level measurements. Where these past analyte level measurements were obtained from a sensor that was no longer calibrated, the past analyte level measurements are not accurate and are offset relative to the values they should be. The flowchart 400 of Figure 4A depicts illustrative steps that may be performed by exemplary embodiments to correct the past analyte measurement values. Initially, a calibrated analyte level measurement is received at step 402. The calibrated analyte level measurement is known to be from a calibration sensor (106A) other than the sensor106 (e.g., other than a CGM that had been providing measurement values) and is known to be properly calibrated, or more calibrated than sensor 106 such that it provides more accurate measurements than sensor 106.

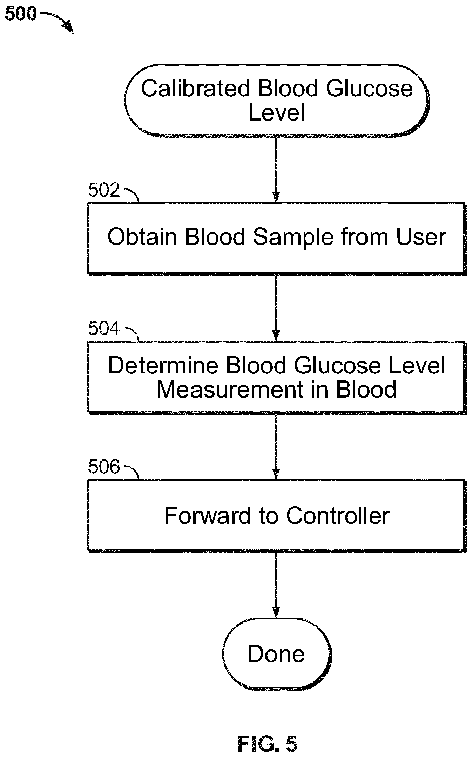

Figure 5 depicts a flowchart 500 of illustrative steps that may be performed to obtain a (more) calibrated (or more accurate) analyte level measurement where the analyte is blood glucose. First at step 502, a blood sample is obtained, such as from a finger prick where the sample is obtained on an enzyme-coated strip. At step 504, the blood glucose concentration is determined by a blood glucose monitor that includes a transducer that converts the enzyme activity with the blood glucose on the enzyme-coated strip to an electrical signal indicating the amount of blood glucose in the sample. Since the calibration sensor (106A) is using blood directly rather than interstitial fluid, for example, as used by a CGM, the blood glucose measurements are typically more accurate than a CGM. The calibrated blood glucose concentration is then forwarded to the controller at step 506.

With respect to Figure 4A, after the calibrated analyte level measurement is received at step 402, the offset between the calibrated analyte level measurement and a recent analyte level measurement (such as the most recent) from the sensor is determined at step 404. Past analyte level measurements stored in the histories used by the controller 110 are updated to correct the offset at step 406. The past analyte level measurements for only a past interval before the properly calibrated analyte level measurement was obtained from the calibration sensor 106A (such as the past 1, 2, 3, 4, 5, 6, 8, 10, 12, 14, 16, or 18 cycles) may be updated in some embodiments. The magnitude of this past interval may be adjustable.

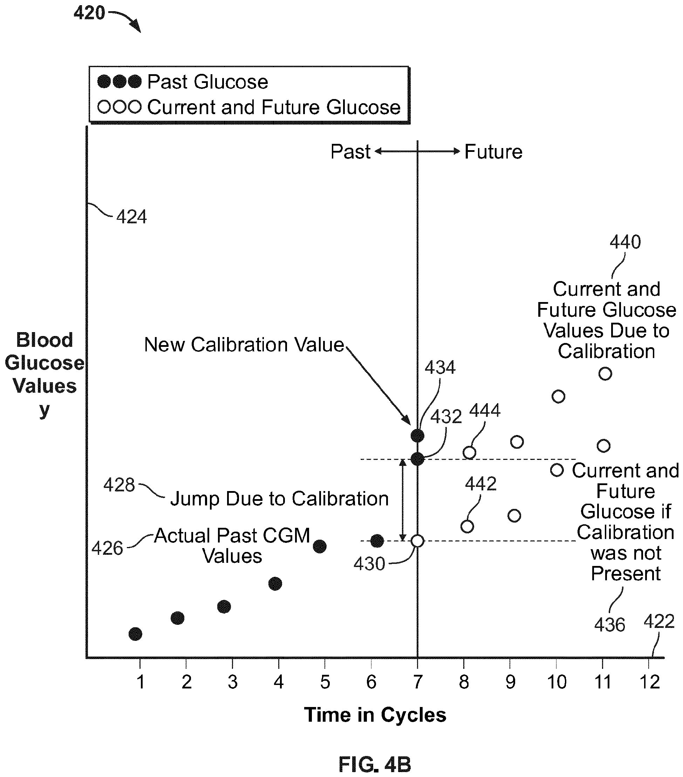

Figure 4B illustrates an example of using a calibrated analyte level measurement to adjust past analyte level measurements. Figure 4B shows a plot 420 of blood glucose concentration values over time. The y axis 424 is the blood glucose concentration, and the x axis 422 is time in cycles (cycle number). In this example, the plot 420 of past blood glucose concentration measurements 426 was obtained from a sensor (in this case a CGM) for cycles 1 through 6. The newly obtained calibrated blood glucose concentration value 434 is obtained at or around or during cycle 7. In this example, each cycle represents 5 minutes. It is determined that the offset due to the lack of proper calibration is of a magnitude that extends from points 430 and 432. The offset is the difference between the current blood glucose concentration reading from the sensor 106 and the current blood glucose concentration from the calibration sensor. The plot 420 also depicts the estimated future blood glucose concentration values 436 or trend if the offset is not applied (i.e., the estimated future values from the sensor). The plot also shows the adjusted estimates of future blood glucose concentration values 440 if the offset is applied. Thus, the offset is applied to the estimated value at 442 at cycle 8 to yield an adjusted estimated value at point 444.

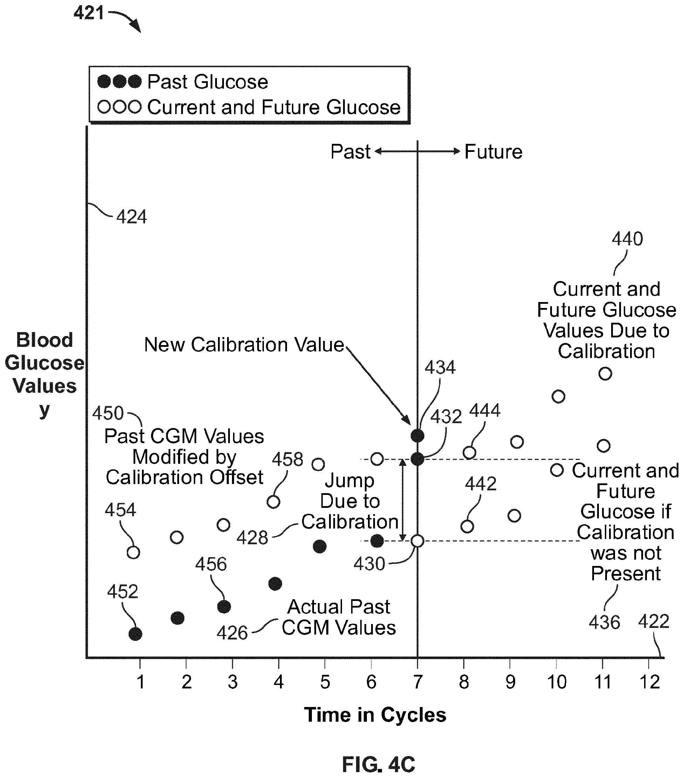

Figure 4C shows a plot 421 like Figure 4B except that the plot 420 of Figure 4C shows the past analyte level measurements (i.e., the past blood glucose concentration measurements adjusted by the offset (see 450). Thus, for example, the offset 428 is added to the blood glucose concentration value 452 to produce adjusted blood glucose concentration value 454. Similarly, the offset 428 is added to past blood glucose concentration value 456 to produce adjusted blood glucose concentration value 458. As was mentioned above, the adjusted blood glucose concentration values 450 are stored in the histories 113 in the storage 114 of the delivery device 102 or in other storage locations accessible by the controller 110 or the processor 119. The adjustments correct for the past loss of calibration. The correction yields more accurate past blood glucose concentration values. The more accurate past blood glucose concentration values, in turn, are used by the controller 110 or processor 199 to produce more accurate predicted future blood glucose concentration values. The more accurate predicted future blood glucose concentration values are relied upon in determining insulin dosages by the controller 110 or processor 119. As a result, dosage values may be generated that are better suited for the actual blood glucose concentration value trajectories, and these dosage values may be adjustments (increase or decrease) to basal dosages, or delivery of a bolus dosage to correct an increasing trend in blood glucose revealed as a result of the calibration and incorporation of the offset 428.

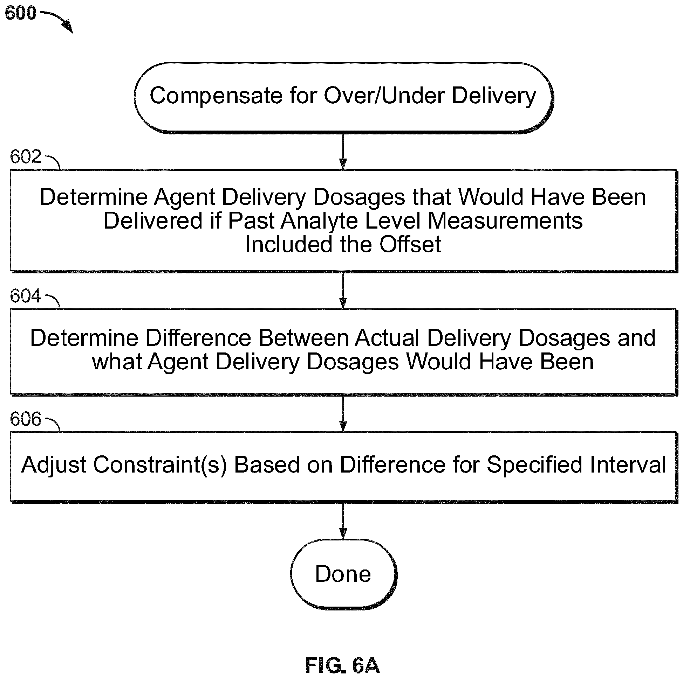

The exemplary embodiments may also account for the past over-delivery or under-delivery of the agent due to the loss of calibration of the sensor 106. This enables the controller 110 or processor 119 to make adjustments to compensate for the over-delivery or under-delivery. Figure 6A depicts a flowchart of illustrative steps that may be performed in exemplary embodiments to compensate for the over-delivery or under-delivery. The first step is to determine what the agent delivery dosages would have been if the past analyte level measurements included the offset at step 602. In other words, determine what the past analyte level measurements would have been if the sensor 106 was properly calibrated. This entails using the adjusted past analyte measurements (e.g., 450 in Figure 4C) in the control algorithm to generate dosage amounts. These predicted dosage amounts are compared to the actual dosage amounts delivered to determine dosage amount differences at step 604. Based on this difference, one or more constraints are adjusted in the control algorithm of the controller 100 or processor 119 to compensate for the difference in dosages at step 606 over a future interval, such as fixed number of cycles (e.g., 1, 2, 3, 4, 5, 6, 8, 10, 12, 14, 16, or 18 future cycles) that is sufficient in length to compensate for the over-delivery or under-delivery of the agent. For example, suppose that the agent is insulin. If too much insulin was delivered due to the loss of calibration, the constraints may be adjusted to deliver less insulin over the future interval. Conversely, if too little insulin was delivered due to the loss of calibration, the constraints may be adjusted to deliver more insulin during the future interval. Alternatively, the basal parameters may be adjusted up or down to encourage or discourage insulin delivery. For example, the basal dosage may be adjusted or the blood glucose concentration target may be adjusted.

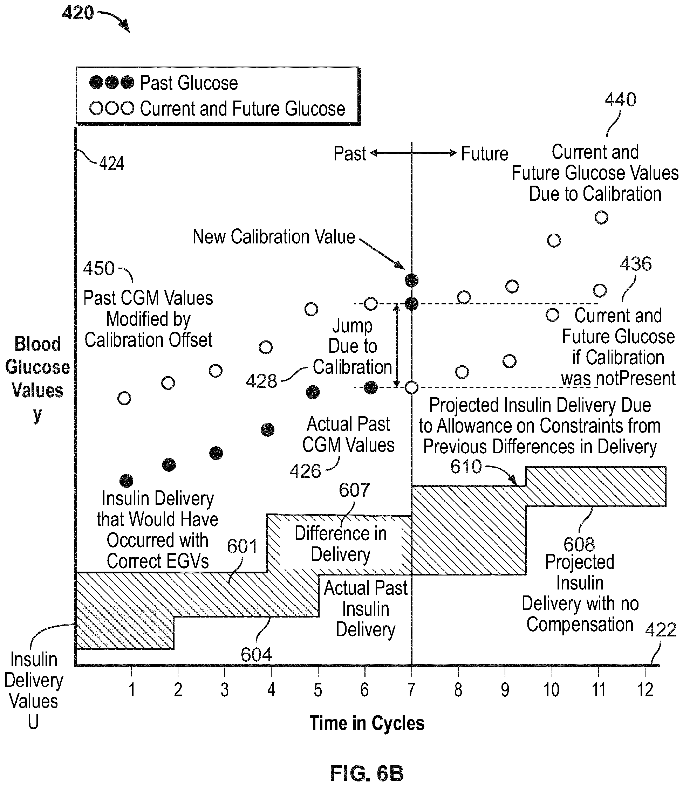

Figure 6B shows an example of insulin delivery for the plot 420 discussed above. Specifically, 604 represents the past insulin delivery dosages that were determined from the past blood glucose measurements 426 from the sensor 106, which was uncalibrated, and 601 represents the insulin dosages that would have been delivered if the offset (corrected) blood glucose concentration measurements 450 were used to determine the dosages (see step 602 in Figure 6A). The difference 607 in dosages is shown as well in Figure 6B (see step 604 in Figure 6A). Figure 6B also shows the projected insulin delivery curve 608 with no compensation for over-delivery or under-delivery. Lastly, Figure 6B, depicts the curve 610 for projected insulin delivery after relaxation of at least one constraint to compensate for past over-delivery or under-delivery of insulin. As can be seen in Figure 6B, for the depicted example, there was an under-delivery of insulin, so the relaxation of the one or more constraints results in an increased delivery of insulin that decreases over time as the additional delivered insulin reduces the blood glucose concentration of the user (see step 606 in Figure 6A). At the end of the future interval, the under-delivery or over-delivery should be fully compensated for, or at least substantially compensated for.

An example of a constraint or element that may be adjusted is the insulin on board (IOB). Insulin on board is the insulin already delivered to the user that still has insulin action to reduce the blood glucose concentration over the user over a specified time window. The insulin to be delivered *I* may be expressed as a formula:

$$I = \frac{G - SP}{CF} - \text{IOB}$$

, where G is the blood glucose concentration, SP is the setpoint for the blood glucose concentration, CF is correction factor which could be estimated by 1800/TDI (total daily insulin).

The controller 110 or processor executing the control application 116 subtracts out the IOB of the user 108 in determining a basal dosage for the user 108 to be delivered by the delivery device 102. Adjusting the IOB value may immediately increase or decrease the dosage that is determined by the control application 116.

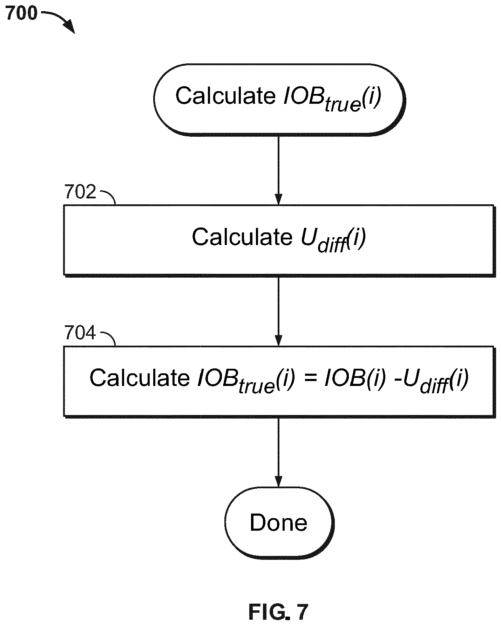

Figure 7 depicts a flowchart 700 of illustrative steps that may be performed by exemplary embodiments in adjusting the IOB constraint. The adjusted IOB value, designated as $IOB_{true}(i)$, for the true IOB value at cycle $i$ is calculated to relax or tighten the constraint to compensate for past over-delivery or under-delivery of insulin. As part of the calculation, a difference designated as $U_{diff}(i)$ is calculated at step 702. This difference $U_{diff}(i)$ captures the difference in actual insulin delivered at cycle $i$ for the previous j cycles and the insulin that would have been delivered if the offset was applied (see the difference 606 in Figure 6B).

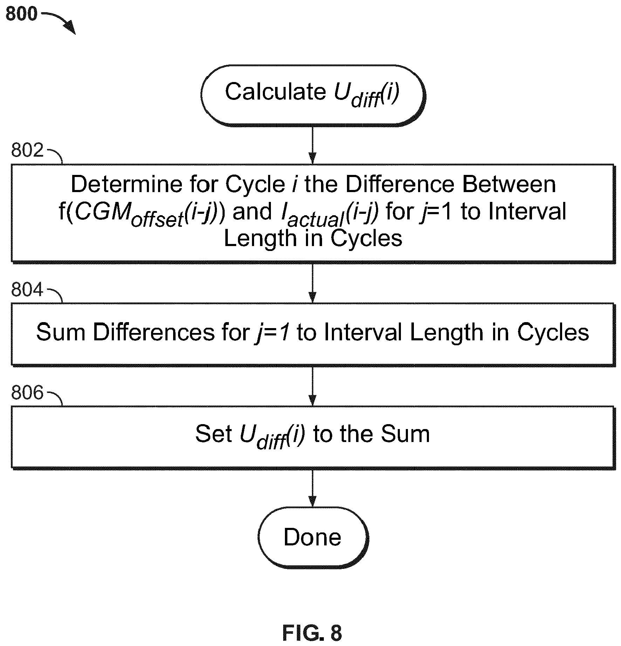

Figure 8 depicts a flowchart 800 of steps that may be performed to determine $U_{diff}(i)$. For the cycle $i$, the difference between the insulin that would have been delivered if the offset was added to the blood glucose concentration reading provided by the sensor 106 and the actual insulin delivered is calculated for each of the preceding $j$ cycles (i.e., the past interval) at step 802. These differences are then summed at step 804. $U_{diff}(i)$ for cycle $i$ is set as the sum at step 806. Thus, $U_{diff}(i)$ is the amount of over-delivery or under-delivery of insulin due to the loss of calibration over the past interval.

With reference to Figure 7, after determining $U_{diff}(i)$ in step 702, the control application 116 subtracts $U_{diff}(i)$ from the IOB at cycle $i$ (i.e., $IOB (i)$) to calculate $IOB_{true}(i)$ at step704. If $U_{diff}(i)$ is negative, then the impact on $IOB_{true}(i)$ is positive, which would cause a decrease in the dosage that is determined by control application 116. And if $U_{diff}(i)$ is positive, then the impact on $IOB_{true}(i)$ is negative, which would cause an increase in the dosage that is determined by control application 116. The value of $U_{diff}(i)$ decays to zero over time, preferably by the end of the future interval during which the control application 116 fully compensates for the past over-delivery or under-delivery in dosages and accordingly, the difference between $IOB_{true}(i)$ and $IOB(i)$_decays to zero over time.

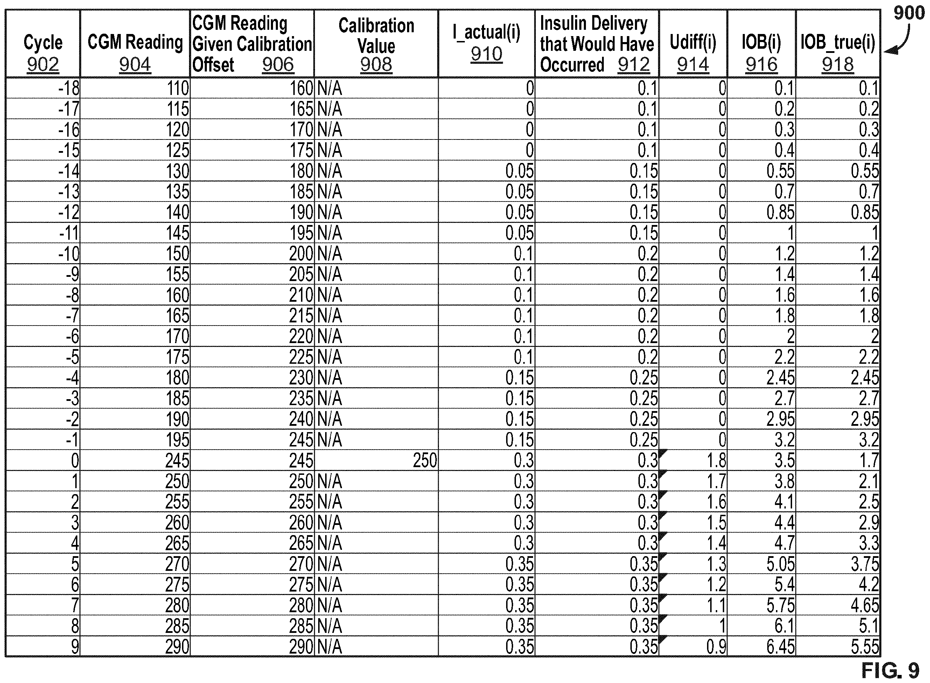

Figure 9 depicts a table 900 of values that help illustrate the effects of the adjustments to past blood glucose concentration values and adjustments to the IOB constraint to compensate for past over-delivery or under-delivery of insulin. The table 900 includes a cycle column 902 that identifies the cycle of the corresponding row. Cycle 0 is when the calibration is made. Negative cycles are cycles that precede cycle 0 and positive cycles are those that follow cycle 0. Thus, cycle -18 is the eighteenth cycle before cycle 0. In this example, j may equal 18.

Column 904 shows the blood glucose concentration measurement for the associated cycle. Column 906 shows the adjusted blood glucose concentration measurement after the offset is added. In this example, the offset is +50. Column 908 shows a calibration value. In this example, only cycle 0 has a calibration value, 250, indicating that the calibration value was obtained during cycle 0. Column 910 shows the $I_{actual}(i)$ value for the cycle, and column 912 holds the insulin delivery dosage that would have been delivered if the offset had been applied. Column 914 holds $U_{diff}(i)$. $U_{diff}(i)$ is zero for the negative numbered cycle as it is not calculated until the calibration occurs. Column 916 shows the $IOB(i)$ values, and column 918 shows the $IOB_{true}(i)$ values.

In this example, the insulin was under-delivered during cycles -18 to -1 due to the loss of calibration. At cycle 0, the calibration value is obtained by the system or input by the user into the system, the offset is applied, and the IOB constraint is adjusted. More insulin is delivered during cycles 1-8 due to the adjustment of the IOB constraint. As can be seen in column 910, the insulin delivered at cycle 0 is 0.3 units rather than the previous 0.15 units, and the insulin delivered increases up to 0.35 units during cycles 5-9. Also, it is seen the $U_{diff}(i)$ decreases from 1.8 at cycle 0 to 0.9 at cycle 9. This is due to the increased insulin delivery due to the adjustment of the IOB constraint.

While the exemplary embodiments have been described herein, various changes in form and detail may be made relative to the exemplary embodiments without departing from the scope of the appended claims appended hereto.

Although the present invention is defined in the attached claims, it should be understood that the present invention can also (alternatively) be defined in accordance with the following embodiments:

    1. A method performed by a processor of an electronic device, comprising:

        receiving a calibrated value for an analyte measurement of a user of a delivery device;
        determining a difference between the calibrated value and a recently received analyte measurement that was received from a sensor;
        updating at least some of past analyte measurements received over a past interval from the sensor based on the difference; and
        using the updated past analyte measurements to determine a new dosage of an agent to be delivered to the user by the delivery device.

    2. The method of embodiment 1, wherein the analyte measurement is a measurement of blood glucose.

    3. The method of embodiment 2, wherein the agent is one of insulin, glucagon, GLP-1 or another agent for modifying the blood glucose of the user.

    4. The method of embodiment 1, wherein the agent includes at least one of a chemotherapeutic agent, a pain relief agent, a blood thinner agent, glucagon, a hormonal agent, a pharmaceutical agent or a therapeutic agent.

5. The method of embodiment 1, wherein the updating comprises updating each of the past analyte measurements that are updated by a same fixed amount.

6. The method of embodiment 1, wherein the electronic device is one of the delivery device or a management device for the delivery device.

7. A method performed by a processor of an electronic device, comprising:

receiving a calibrated value for an analyte measurement of a user of a delivery device that delivers dosages of an agent;
determining a difference between the calibrated value for the analyte measurement and a recently received analyte measurement that was received from a sensor;
updating at least some of past analyte measurements received over a past interval from the sensor based on the difference;
determining dosages of an agent that would have been delivered if the updated past analyte measurements were used to determine the dosages rather than the past analyte measurements received from the sensor;
calculating a difference between the determined dosages of the agent and actual dosages of the agent delivered to the user by the delivery device based on past analyte measurements received for the past interval; and
modifying control for the delivery device to compensate for the calculated difference between the determined dosages of the agent and the actual dosages of the agent.

8. The method of embodiment 7, wherein the agent is insulin, glucagon, GLP-1 or an agent that modifies blood glucose.

9. The method of embodiment 7, wherein the delivery device is an on-body insulin pump.

10. The method of embodiment 7, wherein the analyte is blood glucose.

11. The method of embodiment 7, wherein the agent includes at least one of a chemotherapeutic agent, a pain relief agent, a blood thinner agent, glucagon, a hormonal agent, a pharmaceutical agent or a therapeutic agent.

12. The method of embodiment 7, wherein the calibrated value for the analyte measurement is a blood glucose level measurement resulting from analyzing blood extracted from the user via a different measurement mechanism than the sensor.

13. The method of embodiment 7, wherein the processor uses insulin on board for the user as a factor in determining a dosage of agent to be delivered to the user and wherein the modifying the control for the delivery device to compensate for the calculated difference between the determined dosages of the agent and the actual dosages of the agent comprises updating a value of insulin on board for the user by the difference.

14. The method of embodiment 13, wherein the insulin action of the insulin on board over a time period is used to determine the dosage of agent to be delivered to the user.

15. The method of embodiment 7, wherein the modifying modifies one or more constraints and wherein the modified one or more constraints remain modified for a predetermined period of time of future operation of the delivery device.

16. The method of embodiment 7, wherein the modifying the control for the delivery device to compensate for the calculated difference between the determined dosages of the agent and the actual dosages of the agent further comprises modifying a value of insulin on board for the user.

17. A device for controlling deliveries of a delivery device to a user, the device comprising:

a storage for storing a history of agent dosages delivered to a user and a history of analyte measurements for a user received from a sensor; and
a processor configured for:

receiving a calibrated value for an analyte measurement of a user of the delivery device;
determining a difference between the calibrated value for the analyte measurement and a recently received analyte measurement that was received from a sensor;
updating at least some of past analyte measurements received over a past interval from the sensor based on the difference; and
using the updated past analyte measurements to determine a new dosage of an agent to be delivered to the user by the delivery device.

18. The device of embodiment 17, wherein the processor is further configured for:

determining dosages of the agent that would have been delivered if the updated past analyte measurements were used to determine the dosages rather than the past analyte measurements received from the sensor;
calculating a difference between the determined dosages of the agent and actual dosages of the agent delivered to the user by the delivery device based on past analyte measurements received for the past interval; and
modifying control for the delivery device to compensate for the calculated difference between the determined dosages of the agent and the actual dosages of the agent.

19. The delivery device of embodiment 17, wherein the processor uses insulin on board for the user as a factor in determining a dosage of agent to be delivered to the user and wherein the modifying the control for the delivery device to compensate for the calculated difference between the determined dosages of the agent and the actual dosages of the agent comprises updating a value of insulin on board for the user by the difference.

20. The device of embodiment 17, wherein the analyte measurement is a measurement of blood glucose.

**Claims**

1. A method performed by a processor of an electronic device, comprising:

    receiving a calibrated value for an analyte measurement of a user of a delivery device;
    determining a difference between the calibrated value and a recently received analyte measurement that was received from a sensor;
    updating at least some of past analyte measurements received over a past interval from the sensor based on the difference; and
    using the updated past analyte measurements to determine a new dosage of an agent to be delivered to the user by the delivery device.

2. The method of claim 1, wherein the analyte measurement is a measurement of glucose.

3. The method of claim 2, wherein the agent is one of insulin, glucagon, GLP-1 or another agent for modifying the blood glucose of the user.

4. The method of claim 1, wherein the agent includes at least one of a chemotherapeutic agent, a pain relief agent, a blood thinner agent, glucagon, a hormonal agent, a pharmaceutical agent or a therapeutic agent.

5. The method of claim 1, wherein the updating comprises updating each of the past analyte measurements that are updated by a same fixed amount.

6. The method of claim 5, further comprising determining the fixed amount to update the past analytes measurements from the determined difference between the calibrated value and the recently received analyte measurement that was received from the sensor.

7. The method of claim 1, wherein the electronic device is one of the delivery device or a management device for the delivery device.

8. The method of claim 1, wherein the sensor is a continuous glucose monitor.

9. A device for controlling deliveries of a delivery device to a user, the device comprising:

    a storage for storing a history of agent dosages delivered to a user and a history of analyte measurements for a user received from a sensor; and
    a processor configured for:

        receiving a calibrated value for an analyte measurement of a user of the delivery device;
        determining a difference between the calibrated value for the analyte measurement and a recently received analyte measurement that was received from a sensor;
        updating at least some of past analyte measurements received over a past interval from the sensor based on the difference; and
        using the updated past analyte measurements to determine a new dosage of an agent to be delivered to the user by the delivery device.

10. The device of claim 9, wherein the processor is further configured for:

    determining dosages of the agent that would have been delivered if the updated past analyte measurements were used to determine the dosages rather than the past analyte measurements received from the sensor;
    calculating a difference between the determined dosages of the agent and actual dosages of the agent delivered to the user by the delivery device based on past analyte measurements received for the past interval; and
    modifying control for the delivery device to compensate for the calculated difference between the determined dosages of the agent and the actual dosages of the agent.

11. The device of claim 9, wherein the processor uses insulin on board for the user as a factor in determining a dosage of agent to be delivered to the user and wherein the modifying the control for the delivery device to compensate for the calculated difference between the determined dosages of the agent and the actual dosages of the agent comprises updating a value of insulin on board for the user by the difference.

**12.** The device of claim 9, wherein the analyte measurement is a measurement of glucose.

**13.** The device of claim 9, wherein the device is one of the delivery device or a management device for the delivery device.

**14.** The device of claim 9, wherein the agent is one of insulin, glucagon, GLP-1 or another agent for modifying the blood glucose of the user.

**15.** The device of claim 9, wherein the updating comprises updating each of the past analyte measurements that are updated by a same fixed amount.

**FIG. 1A**

150

152 Agent

154 Insulin
156 Glucagon
158 GLP
160 Hormonal Agent

162 Blood Thinner
164 Chemotherapy Agent
166 Pain Relief Agent
168 Antibiotic
170 Anti-Depressant

172 Therapeutic Agent
174 Pharmaceutical Agent

**FIG. 1B**

FIG. 2

300

Control Loop

302
Obtain Analyte Measurement at Sensor

304
Send Measurement to Controller

306
Calculate Difference Between
Measurement and Target at Controller

308
Apply Function to Select Dosage

310
Send Control Signal to Dispense Dosage

Done

FIG. 3

400

Adjust Past Analyte
Measurements

402

Receive Calibrated Analyte Measurement

404

Determine Offset Between Calibrated
Analyte Measurement and Recent Analyte
Measurement from Sensor

406

Adjust Past Analyte Measurement History
for an Interval Based on Offset

Done

**FIG. 4A**

420

● ● ● Past Glucose
○ ○ ○ Current and Future Glucose

Past ◄────► Future

424

440

Current and
Future Glucose
Values Due to
Calibration
○

Blood
Glucose
Values
y

New Calibration Value

434

○

432 444

●

○ ○

○ Current and
Future
Glucose if
Calibration
was not
Present

428

Jump Due to Calibration

442

○

426

Actual Past CGM
Values

●

● ○

○

430

436  422

1   2   3   4   5   6   7   8   9   10  11  12

**Time in Cycles**

**FIG. 4B**

**FIG. 4C**

500

Calibrated Blood Glucose
Level

502

Obtain Blood Sample from User

504

Determine Blood Glucose Level
Measurement in Blood

506

Forward to Controller

Done

FIG. 5

600

Compensate for Over/Under Delivery

602 —

Determine Agent Delivery Dosages that Would Have Been Delivered if Past Analyte Level Measurements Included the Offset

604 —

Determine Difference Between Actual Delivery Dosages and what Agent Delivery Dosages Would Have Been

606 —

Adjust Constraint(s) Based on Difference for Specified Interval

Done

FIG. 6A

FIG. 6B

700 ⟍

Calculate $IOB_{true}(i)$

702 ⟍

Calculate $U_{diff}(i)$

704 ⟍

Calculate $IOB_{true}(i) = IOB(i) - U_{diff}(i)$

Done

**FIG. 7**

$800$

Calculate $U_{diff}(i)$

802 — Determine for Cycle $i$ the Difference Between $f(CGM_{offset}(i\text{-}j))$ and $I_{actual}(i\text{-}j)$ for $j$=1 to Interval Length in Cycles

804 — Sum Differences for $j$=1 to Interval Length in Cycles

806 — Set $U_{diff}(i)$ to the Sum

Done

FIG. 8

| Cycle 902 | CGM Reading 904 | CGM Reading Given Calibration Offset 906 | Calibration Value 908 | I_actual(i) 910 | Insulin Delivery that Would Have Occurred 912 | Udiff(i) 914 | IOB(i) 916 | IOB_true(i) 918 |
|---|---|---|---|---|---|---|---|---|
| -18 | 110 | 160 | N/A | 0 | 0.1 | 0 | 0.1 | 0.1 |
| -17 | 115 | 165 | N/A | 0 | 0.1 | 0 | 0.2 | 0.2 |
| -16 | 120 | 170 | N/A | 0 | 0.1 | 0 | 0.3 | 0.3 |
| -15 | 125 | 175 | N/A | 0 | 0.1 | 0 | 0.4 | 0.4 |
| -14 | 130 | 180 | N/A | 0.05 | 0.15 | 0 | 0.55 | 0.55 |
| -13 | 135 | 185 | N/A | 0.05 | 0.15 | 0 | 0.7 | 0.7 |
| -12 | 140 | 190 | N/A | 0.05 | 0.15 | 0 | 0.85 | 0.85 |
| -11 | 145 | 195 | N/A | 0.05 | 0.15 | 0 | 1 | 1 |
| -10 | 150 | 200 | N/A | 0.1 | 0.2 | 0 | 1.2 | 1.2 |
| -9 | 155 | 205 | N/A | 0.1 | 0.2 | 0 | 1.4 | 1.4 |
| -8 | 160 | 210 | N/A | 0.1 | 0.2 | 0 | 1.6 | 1.6 |
| -7 | 165 | 215 | N/A | 0.1 | 0.2 | 0 | 1.8 | 1.8 |
| -6 | 170 | 220 | N/A | 0.1 | 0.2 | 0 | 2 | 2 |
| -5 | 175 | 225 | N/A | 0.1 | 0.2 | 0 | 2.2 | 2.2 |
| -4 | 180 | 230 | N/A | 0.15 | 0.25 | 0 | 2.45 | 2.45 |
| -3 | 185 | 235 | N/A | 0.15 | 0.25 | 0 | 2.7 | 2.7 |
| -2 | 190 | 240 | N/A | 0.15 | 0.25 | 0 | 2.95 | 2.95 |
| -1 | 195 | 245 | N/A | 0.15 | 0.25 | 0 | 3.2 | 3.2 |
| 0 | 245 | 245 | 250 | 0.3 | 0.3 | 1.8 | 3.5 | 1.7 |
| 1 | 250 | 250 | N/A | 0.3 | 0.3 | 1.7 | 3.8 | 2.1 |
| 2 | 255 | 255 | N/A | 0.3 | 0.3 | 1.6 | 4.1 | 2.5 |
| 3 | 260 | 260 | N/A | 0.3 | 0.3 | 1.5 | 4.4 | 2.9 |
| 4 | 265 | 265 | N/A | 0.3 | 0.3 | 1.4 | 4.7 | 3.3 |
| 5 | 270 | 270 | N/A | 0.35 | 0.35 | 1.3 | 5.05 | 3.75 |
| 6 | 275 | 275 | N/A | 0.35 | 0.35 | 1.2 | 5.4 | 4.2 |
| 7 | 280 | 280 | N/A | 0.35 | 0.35 | 1.1 | 5.75 | 4.65 |
| 8 | 285 | 285 | N/A | 0.35 | 0.35 | 1 | 6.1 | 5.1 |
| 9 | 290 | 290 | N/A | 0.35 | 0.35 | 0.9 | 6.45 | 5.55 |

900

FIG. 9

EP 4 075 439 A1

EUROPEAN SEARCH REPORT

Europäisches Patentamt
European Patent Office
Office européen des brevets

Application Number

EP 22 16 8389

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/298765 A1 (BUDIMAN ERWIN S [US] ET AL) 25 November 2010 (2010-11-25) | 1-4,7-15 | INV. G16H20/10 |
| Y | * [0005] – [0006], [0010], [0053] – [0054] * | 5,6 | |
| Y | WO 2014/163815 A1 (EDWARDS LIFESCIENCES CORP [US]) 9 October 2014 (2014-10-09) | 5,6 | |
| A | * par 12 * | 1-4,7-15 | |
| A | DEL FAVERO SIMONE ET AL: "Retrofitting of Continuous Glucose Monitoring Traces Allows More Accurate Assessment of Glucose Control in Outpatient Studies", DIABETIS TECHNOLOGY & THERAPEUTICS, [Online] vol. 17, no. 5, 1 May 2015 (2015-05-01), pages 355-363, XP055937085, MARY ANN LIEBERT, LARCHMONT, NY; US ISSN: 1520-9156, DOI: 10.1089/dia.2014.0230 Retrieved from the Internet: URL:https://www.liebertpub.com/doi/pdf/10.1089/dia.2014.0230> [retrieved on 2022-06-30] * page 355 left col, page 357 right col last par – page 358 right col penultimate par * | 1-15 | TECHNICAL FIELDS SEARCHED (IPC) G16H |

The present search report has been drawn up for all claims

| Place of search | Date of completion of the search | Examiner |
|---|---|---|
| Munich | 29 August 2022 | Bankwitz, Robert |

EPO FORM 1503 03.82 (P04C01)

## EP 4 075 439 A1

### ANNEX TO THE EUROPEAN SEARCH REPORT
### ON EUROPEAN PATENT APPLICATION NO.

EP 22 16 8389

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2022

| Patent document cited in search report | | Publication date | Patent family member(s) | | Publication date |
|---|---|---|---|---|---|
| US 2010298765 | A1 | 25-11-2010 | EP | 2433235 A2 | 28-03-2012 |
| | | | US | 2010298765 A1 | 25-11-2010 |
| | | | US | 2012330227 A1 | 27-12-2012 |
| | | | US | 2014180203 A1 | 26-06-2014 |
| | | | US | 2019054237 A1 | 21-02-2019 |
| | | | US | 2020197607 A1 | 25-06-2020 |
| | | | US | 2022047812 A1 | 17-02-2022 |
| | | | WO | 2010135654 A2 | 25-11-2010 |
| WO 2014163815 | A1 | 09-10-2014 | NONE | | |

EPO FORM P0459

For more details about this annex : see Official Journal of the European Patent Office, No. 12/82

26